Europäisches Patentamt

European Patent Office

Office européen des brevets

(11) Veröffentlichungsnummer: **0 307 390**
**A2**

## (12) EUROPÄISCHE PATENTANMELDUNG

(21) Anmeldenummer: **88890226.9**

(22) Anmeldetag: **02.09.88**

(51) Int. Cl.⁴: **A 61 K 31/40**
**C 12 P 13/04**

(30) Priorität: **04.09.87 AT 2235/87   07.04.88 AT 898/88**

(43) Veröffentlichungstag der Anmeldung:
**15.03.89 Patentblatt 89/11**

(84) Benannte Vertragsstaaten:
**AT BE CH DE ES FR GB GR IT LI LU NL SE**

(71) Anmelder: **MERCKLE GMBH**
**Graf-Arco-Strasse 3**
**D-7900 Ulm (DE)**

**Lubec, Gert Dr.,**
**Schmalzhofgasse 18/3/5**
**A-1060 Wien (AT)**

(72) Erfinder: **Lubec, Gert, Dr.**
**Schmalzhofgasse 18/3/5**
**A-1060 Wien (AT)**

(74) Vertreter: **Atzwanger, Richard, Dipl.-Ing.**
**Mariahilfer Strasse 1c**
**A-1060 Wien (AT)**

(54) **Chemische Verbindung bestehend aus cis-3-hydroxyprolin zur therapeutischen Behandlung und Verfahren zu deren Herstellung.**

(57) Beschrieben wird die Anwendung von cis-3-Hydroxyprolin in einem Verfahren zur Bekämpfung von Verdickungen der Basalmembran.

Weiters werden Arzneimittel und pharmazeutische Zubereitungen, die als Wirkstoff cis-3-Hydroxyprolin enthalten, angegeben.

cis-3-Hydroxyprolin kann auch bei der Herstellung eines Arzneimittels zur Verwendung bei der Bekämpfung von Verdickungen der Basalmembran eingesetzt werden.

EP 0 307 390 A2

Beschreibung

## Chemische Verbindung bestehend aus cis-3-Hydroxyprolin zur therapeutischen Behandlung und Verfahren zu deren Herstellung

Die Erfindung betrifft eine chemische Verbindung, bestehend aus cis-3-Hydroxyprolin zur Anwendung in einem Verfahren zur therapeutischen Behandlung des menschlichen oder tierischen Körpers, insbesondere zur Anwendung bei der Bekämpfung von Verdickungen der Basalmembran.

Die Erfindung betrifft weiters Arzneimittel, die aus cis-3-Hydroxyprolin bestehen.

Die Erfindung erstreckt sich auch auf pharmazeutische Zubereitungen, die dadurch gekennzeichnet sind, daß sie als Wirkstoff cis-3-Hydroxyprolin sowie einen inerten pharmazeutischen Träger enthalten.

Schließlich umfaßt die Erfindung auch die Verwendung von cis-3-Hydroxyprolin bei der Herstellung eines Arzneimittels zur Verwendung bei der Bekämpfung von Verdickungen der Basalmembran.

Bei der Herstellung von 3-Hydroxyprolin durch chemische Synthese oder durch Gewinnung aus natürlichen Quellen (z.B. Kadaververwertung usw.) fallen die beiden isomeren Formen, nämlich cis-3-Hydroxyprolin und trans-3-Hydroxyprolin in wechselnden Mengenanteilen an.

Die grundsätzlichen Möglichkeiten für die Synthese bzw. Isolierung von 3-Hydroxyprolin (Gemisch der cis- und der trans-Form) sind

a) Synthese aus Aminomalonsäure (in der Regel eingesetzt als Diäthylaminomalonsäure) und Acrolein,

b) Synthese von cis- und trans-3-Phenoxyprolin nach J. Häusler and U. Schmidt in Liebigs Annalen der Chemie 1979, 1881 - 1889,

c) Darstellung von cis- und trans-C-3-substituierten Prolinverbindungen von J. Häusler in Liebigs Annalen der Chemie 1981, 1073 - 1088,

d) Isolierung aus bei der Kadaververwertung anfallenden, hydrolisierten Kollagenen,

e) Isolierung aus Meeresschwämmen und dgl. mehr.

Die oben geschilderten Möglichkeiten der Gewinnung von 3-Hydroxyprolin haben u. a. den Nachteil, daß stets ein Gemisch der cis- und der trans-Form anfällt und daß teilweise hochgiftige Ausgangssubstanzen (z. B. Acrolein) angewendet werden müssen.

In J. Häusler-Darstellung von cis- und trans-C-3-substituierten Prolinverbindungen (Liebigs Annalen der Chemie 1981, 1073-1088) ist u. a. der Vorschlag enthalten, cis-3-Hydroxyprolin aus 3-Acetyloxyprolin durch Hydrolyse mit Salzsäure herzustellen.

J. Häusler berichtet a.a.O. auch, daß sich die Natrium-1-pyrrolin-2-carboxylate mit Hilfe von Natriumborhydrid zu den ciskonfigurierten Prolinanaloga, so u. a. der 3-Acetyloxyverbindung reduzieren lassen. Diese Reduktion soll mit hoher Selektivität (über 95 %) erfolgen. Allerdings ist die Reduktion des in der C-3-Position durch eine Hydroxylgruppe substituierten 1.2-Dehydroprolins zum cis-3-Hydroxyprolin von J. Häusler nicht beschrieben worden.

Der Erfindung liegt die Erkenntnis zugrunde, daß cis-3-Hydroxyprolin eine therapeutische Wirksamkeit besitzt und insbesondere bei der Bekämpfung von Verdickungen der Basalmembran wirksam ist.

Bisher ist die therapeutische Wirksamkeit von cis-3-Hydroxyprolin noch nicht beschrieben worden.

Die der Erfindung zugrundeliegende Verbindung kann durch eine Oxidationsreaktion des trans-3-Hydroxyprolins zur entsprechenden 1.2-Dehydroverbindung und anschließende Reduktion der so erhaltenen 1.2-Dehydroverbindung (zu cis-3-Hydroxyprolin) hergestellt werden.

Die erfindungsgemäße Substanz, cis-3-Hydroxyprolin ist eine bei Säugern (einschließlich des Menschen) nicht vorkommende Aminosäure. Die zur erfindungsgemäßen Verbindung isomere Verbindung (trans-3-Hydroxyprolin) ist ein wesentlicher, integraler Bestandteil des Basalmembrankollagens. Es ist bekannt, daß dieses Basalmembrankollagen die Gefäßwand bei pathologischen Zuständen, wie z. B. bei Diabetes mellitus verdickt und so zu schweren Gefäßverengungen führt.

Bei Tierversuchen an Maus und Ratte wurde festgestellt, daß das erfindungsgemäße cis-3-Hydroxyprolin, das bislang zur Verwendung als pharmazeutischer Wirkstoff nicht vorgeschlagen worden ist, hier Abhilfe schaffen kann. Es hat sich nämlich überraschenderweise gezeigt, daß durch Einbau des zur trans-Form isomeren cis-3-Hydroxyprolins in Kollagen ein instabiles, minderwertiges und nicht helikales Kollagenmolekül gebildet wird, woraus eine insgesamt verringte Synthese von Basalmembrankollagen resultiert.

Diese Beeinflussung des Kollagenstoffwechsels durch cis-3-Hydroxyprolin wurde durch die weiter unten wiedergegebenen Tierversuche bestätigt. Überdies ist das erfindungsgemäße cis-3-Hydroxyprolin eine Substanz, die Toxizitätsversuchen keine schädliche Wirkung gezeigt hat. Auch eine Teragontenität kann in der ersten Generation ausgeschlossen werden.

Das beim zur Herstellung der erfindungsgemäßen Verbindung angewendeten Verfahren als Ausgangsstoff dienende trans-3-Hydroxyprolin (das gegebenenfalls in Mischung mit cis-3-Hydroxyprolin vorliegen kann) kann auf verschiedene Weisen gewonnen werden.

a) Isolierung von trans-3-Hydroxyprolin aus kommerziell erhältlichen Champignons,

b) Isolierung von trans-3-Hydroxyprolin aus Schwämmen,

c) Synthese eines Gemisches aus cis- und trans-3-Hydroxyprolin aus Aminomalonsäure und Acrolein,

d) Darstellung nach J. Häusler a.a.O. und

e) (erfindungsgemäß bevorzugt) aus den bei der Kadaververwertung durch Hydrolyse anfallenden Aminosäuren.

Die bei der Kadaververwertung nach deren Hydrolyse anfallenden Aminosäuren enthalten als wichtigsten Bestandteil Prolin, das in großen Mengen erzeugt und als Futtermittel Verwendung findet. Je Tonne Prolin fallen etwa 5 bis 10 kg trans-3-Hydroxyprolin, an. Bislang wurde dieses zusammen mit den anderen Aminosäuren verworfen. Das nachstehend beschriebene Verfahren ermöglicht es aber, das trans-3-Hydroxyprolin in großem Maßstab zu cis-3-Hydroxyprolin zu isomerisieren.

Bei der praktischen Ausführung des Verfahrens erfolgt die Oxidation mit Hilfe von Aminosäureoxidasen (D-Aminosäureoxidase und L-Aminosäureoxidase) und Catalase. Da im Gegensatz zur Catalase die Aminosäureoxidasen sehr teuer sind, werden diese beim Verfahren bevorzugt immobilisert eingesetzt.

Die Immobilisierung kann dadurch erfolgen, daß die Enzyme in einem Dialyseschlauch (Hersteller: Union Carbide) eingeschlossen oder an Kügelchen (beads) gekuppelt eingesetzt werden. Diese Kügelchen können beispielsweise aus mit Cyanbromid aktivierter Sepharose 4B (Hersteller: Pharmacia, Uppsala) oder an Affi-Gel-10 (Hersteller: Bio-Rad) bestehen.

Nach 24 Stunden langer Inkubation bei 37°C ist die Oxidation zur 1.2-Dehydroverbindung abgeschlossen und die immobilisierten Enzyme können erneut eingesetzt werden. Beispielsweise können die an beads immobilisierten Enzyme durch einfache Sedimentation oder durch Zentrifugieren aus dem Reaktionsgemisch abgetrennt werden. (Es folgt die Reduktion mit Natriumborhydrid.)

Pharmakologische Eingenschaften des erfindungsgemäßen cis-3-Hydroxyprolins:

a) Teratogenizität:

10 weibliche, durch Inzucht erzeugte, weiße Mäuse (Österr. Institut für Versuchstierkunde, Himberg) wurden für diesen Versuch verwendet.

Die Schwangerschaft wurde durch die Vaginalpfropftechnik bestimmt. Zum Zeitpunkt der Diagnose der Gravidität wurden sie mit einer 0,1 g/Vol.-% Lösung von cis-3-Hydroxyprolin gefüttert. Die Schwangerschaft verlief im Vergleich zu nicht behandelten, trächtigen Mäusen ereignislos. Es traten keine postpartalen Blutungen auf. Die Gestationsperiode war von adäquater Länge. Die Zahl der Nachkommen war normal. Es wurden keine Änderungen des Verhaltens oder Kannibalismus festgestellt. Die Nachkommen begannen mit dem Saugen kurz nach der Niederkunft.

Die Plazenten hatten eine normale Größe und ein normales Aussehen.

Die Nieren von zehn Tieren wurden im Elektronenmikroskop untersucht. Abgesehen von Proteinsekret-Tröpfchen konnten keine Änderungen beobachtet werden.

Bei der Autopsie getöteter Jungtiere konnten keine makroskopischen Organänderungen gefunden werden. Es konnten auch keine Mißbildungen der Jungtiere festgestellt werden.

Schließlich trat bei der Niederkunft oder danach keine Blutung auf.

b) Bestimmung von Kollagen Typ I:

Kollagen Typ I wurde in 10 Ratten, die mit cis-3-Hydroxyprolin gefüttert wurden und bei 10 Ratten, die als Vergleichstiere dienten, bestimmt. Die verwendeten Tiere waren alle Diabetiker, ganz gleich, ob behandelt oder nicht behandelt.

Die Abtrennung von Kollagen des Typs I wurde nach einer Standardmethode ausgeführt. Das Kollagen wurde hydrolisiert und affinitätschromatografisch bestimmt.

Das Kollagen Typ I, wiedergegeben durch die 4-Hydroxyprolinkonzentrationen, unterschied sich nicht zwischen der mit cis-3-Hydroxyprolin gefütterten Gruppe von Ratten und der Vergleichsgruppe.

Es konnten in den behandelten Tieren 1076 mg 4-Hydroxyprolin und in den nicht behandelten Tieren 1165 mg 4-Hydroxyprolin gefunden werden.

Der Student's t-Test zeigte keine signifikanten Unter schiede zwischen den Gruppen (p kleiner als 0,3).

c) Bestimmung von Kollagen Typ I aus der Haut:

Kollagen Typ I wurde aus der Haut von Mäusen isoliert. Die Versuchstiere wurden in Gruppen zu je 10 Tieren eingeteilt, wovon eine mit cis-3-Hydroxyprolin gefüttert wirde und die andere Gruppe als Vergleichsgruppe diente. Die Abtrennung des Kollagens Typ I erfolgte nach einer Standardmethode.

Das abgetrennte Kollagen wurde auf einer Mikrowaage gewogen. Dadurch wurde festgestellt, daß die Kollagenkonzentrate der nicht behandelten Tiere 575 mg $\pm$ 125 mg wogen, wogegen die Kollagenkonzentrate der mit cis-3-Hydroxyprolin behandelten Versuchstiere im Durchschnitt 539 mg $\pm$ 175 mg betrugen.

Durch Anwendung des Studen's t-Test (p kleiner als 0,4) wurden keine signifikanten Unterschiede festgestellt.

d) Bestimmung von Kollagen Type IV:

Es wurden mit cis-3-Hydroxyprolin behandelte Mäuse und nicht behandelte Mäuse verwendet. Die rechte Niere jedes Versuchstieres wurde für biochemische Bestimmungen und die linke Niere jedes Versuchstieres für Immunflureszenzuntersuchungen herangezogen.

Es wurden zwei Verfahren angewendet:

Die Bestimmung von trans-3- und trans-4-Hydroxyprolin nach dem nachstehend beschriebenen Verfahren und ein affinitätschromatografisches Verfahren unter Verwendung von anti-IV-IgG, gekoppelt an Sepharose oder Agarose-Kügelchen.

Die mit cis-3-Hydroxyprolin behandelten Mäuse zeigten einen abnehmenden Gehalt von Kollagen Typ IV, wobei Pepsinaufschlüsse der Nieren verwendet wurden (p kleiner als 0,01).

Die Bestimmung von trans-3- und trans-4-Hydroxyprolin zeigte signifikante Unterschiede in den Pepsinaufschlüssen der beiden Gruppen, welche anzeigten, das der Kollagen-Typ-IV-Gehalt in den Nieren behandelter Tiere verringert ist (p kleiner als 0,005).

Nachstehend wird das Verfahren zur Bestimmung von trans-3- bzw. trans-4-Hydroxyprolin erläutert:

Die Proben wurden nun durch den Ionenaustauscher (3 ml DOWEX 50 W-X8 von Bio-Rand Nr. 745-6441) geschickt und die aufgefangenen Aminosäuren mit 2 ml von 4 M Ammoniak eluiert, um anschließend unter Stickstoff getrocknet zu werden.

Durch diese Vorbereitung mittels Aktivkohle und Ionenaustausch konnten störende Substanzen eliminiert werden. Die getrockneten Proben wurden dann in 200 µl destilliertem Wasser aufge löst, 100 µl davon wurden mit 20 µl O-Phthaladehyd (8 mg/ml gelöst in 30 % Triäthylamin/70 % Äthanol, Pierce Nr. 26.010) versetzt und für 30 min bei Zimmertemperatur ins Dunkle gestellt; anschließend wurde mit Stickstoff getrocknet. Das gewonnene dunkle, trockene Material wurde in 20 µl destilliertem Wasser gelöst und mit 20 µl 7-Chloro- 4-nitrobenz-2-oxa-1,3-diazol (8 mg/ml Äthanol, Sigma Nr. C 5261) versetzt, danach für 10 min in ein 45°-Wasserbad gestellt und anschließend zentrifugiert. 10 µl dieser Mischung wurden dann auf HPTLC-Platten (LHP'KF von Whatman 4806-710) aufgetragen und mittels Laufmittel aufgetrennt (Laufmittel: Azeton : Chloroform : Methanol ; Triäthylamin = 20:60:12,5:7,5). Bei jeder Platte wurden Standardmischungen bestehend aus:
trans-4-Hydroxyprolin (Sigma H 6002)
cis-4-Hydroxyprolin (Sigma H 1637)
trans-3-Hydroxyprolin (synthetisiert nach Häusler, a.a.O.
cis-3-Hydroxyprolin (synthetisiert nach Häusler) verwendet.

### e) Opthalmologische Untersuchungen:

10 mit cis-3-Hydroxyprolin behandelte Mäuse wurden mit 10 Vergleichstieren verglichen.

Nach einmonatiger Verabreichung von cis-3-Hydroxyprolin an die 10 Mäuse wurden die Augen mit der Spaltlampe untersucht und eine Fundoskopie ausgeführt.

Es konnten keine Unterschiede zwischen der behandelten Gruppe und der nicht behandelten Gruppe von Versuchstieren festgestellt werden.

Eine elektronenmikroskopische Untersuchung zeigte keine morphologischen Änderungen der Augen beider Gruppen.

Es gab weder klinische Hinweise noch Symptome für Sehstörungen.

### f) Überprüfung der Änderung von rasch wachsenden Organen:

### Haare:

es konnten keine Veränderungen der Haare von Mäusen und Sprague-Dawley-Ratten, die mit cis-3-Hydroxyprolin behandelt wurden, festgestellt werden. Bei einer nach dem von G. Lubec in Lancet 1983, 1985 beschriebenen Verfahren zur Infrarotspektroskopie ausgeführten Untersuchung konnten keine strukturellen Änderungen bei Mäusen festgestellt werden.

### Zähne:

Die Festigkeit der Zähne der Mäuse bzw. der Ratten unterschied sich nicht zwischen behandelten und nicht behandelten Tieren. Diese Feststellung wurde durch Röntgenuntersuchungen bestätigt.

Unterschiedliche Färbungen des Blutes zeigten keine quantitativen oder qualitativen Änderungen der lymphocyten oder polymorphonuklearer Zellen, es konnten auch keine Einschlüsse oder Fusionen bzw. Vakuolisierung festgestellt werden.

### g) Hämoglobinänderungen:

Durch Hämoglobinelekrophorese auf Zelluloseacetat konnten keine Unterschiede der Mäusesysteme zwischen behandelten und nicht mit cis-3-Hydroxyprolin behandelten tieren festgestellt werden

### h) Heilung von Wunden:

Zwei Gruppen zu je 8 weißen, weiblichen Ratten wurden für diesen Versuch herangezogen. Ein 10 mg Polyvinylschwamm wurde subkutan 4 Tage lange eingepflanzt. Darauf wurde der Schwamm entfernt und der Gehalt an trans-4-Hydroxyprolin nach dem Prinzip von Kivivikko, K.I., Laitinen, O., Prokop, D.O. in Anal. Biochem. 19, 249 (1967) bestimmt.

Der mittlere Gehalt an 4-Hydroxyprolin in den Schwämmen von 8 Vergleichstieren betrug 1,75 µg. Der durchschnittliche Gehalt an 4-Hydroxyprolin in den mit cis-3-Hydroxyprolin gefütterten Tieren betrug 1,87 µg.

Es gab auch keinen signifikanten Unterschied zwischen den Gruppen, wenn diese nach dem Student's t-Test verglichen werden.

### i) Akute Toxizität:

100 mg cis-3-Hydroxyprolin wurden intravenös 10 Sprague-Dawley-Ratten verabreicht.

1 Tag nach der Verabreichung der Versuchsverbindung und 1 Woche danach wurden Blutproben von 5 Tieren gezogen. Es wurden die Parameter, Creatinin, GOT (Glutamat-Oxalacetat-Transaminase), GPT (Glutamat-Pyruvat-Transaminase), yGT ( - Glutamyl-Transpeptidase), Alkaliphosphatase und die üblichen hämatologischen Parameter bestimmt.

Im Vergleich zu den Vergleichstieren wurden keine signifikanten Unterschiede (p = 0,4) beobachtet.

### j) Bestimmung der Aktivität von Kollagenase in der Niere:

Zehn diabetische Wistar-Ratten wurden mit cis-3-Hydroxyprolin gefüttert und 10 Wistar-Ratten die als Vergleichstiere dienten, gegenübergestellt. Die Nierenhomogenisate wurden hinsichtlich ihrer kollagenolytischen Aktivität nach der Vorschrift von Gries et al bestimmt.

Es konnte keine statistisch signifikanten Änderungen der kollagenolytischen Aktivität zwischen den beiden Gruppen von Versuchstieren festgestellt werden. Daraus folgt, daß die durch die erfindungsgemäße Verbindung bewirkte Reduktion der Basalmembrandicke nicht die Folge einer erhöhten Kollagenolyse ist.

k) Wirksamkeit von cis-3-Hydroxyprolin:

Die Gefäßwand besteht aus Endothel, Epithel und der Basalmembran. Beim Diabetes mellitus kommt es zu einer signifikanten Verbreiterung der Bassalmembran. Diese besteht zu 90 % aus Kollagen Typ IV. Der Angriffspunkt des cis-3-Hydroxyprolin ist am Kollagen Type IV.

Affinitätschromatografische Studien zur Bestimmung von Kollagen Typ IV zeigen eine deutliche, statistisch signifikante Verminderung der pathologischen Basalmembranproliferation.

Diese Beobachtung wurde durch elektronenmikroskopische Untersuchungen untermauert.

Eine signifikante Reduktion der Basalmembrandicke kann nach Therapie mit cis-3-Hydroxyprolin im Unterschied zu einer nichtbehandelten Kontrollgruppe erhoben werden (vgl. oben Pkt. d).

Nachstehend werden Verfahren zur Herstellung der erfindungsgemäßen Verbindung anhand von nicht beschränkenden Beispielen näher erläutert:

Beispiel 1

1 mg trans-3-Hydroxyprolin wird zusammen mit 1 mg Catalase (Catalase aus Rinderleber mit einer spezifischen Aktivität von etwa 65 0000 U/mg, erhältlich bei Boehringer Mannheim), 1 mg D-Aminosäureoxidase (aus Schweinenieren, Hersteller Boehringer Mannheim, spezifische Aktivität etwa 3 U/mg) und mit 0,5 mg L-Aminosäureoxidase (aus krotalus turisus, spezifische Aktivität 3,5 U/mg, Hersteller Boehringer, Mannheim) in 0,025 ml Phsophatpuffer (pH-Wert 8,0) bei 37°C 24 Stunden lang inkubiert. Die eingesetzten Enzyme (Catalase und Aminosäureoxidasen) waren an Kügelchen (beads) aus mit Cyanbromid aktivierter Sepharose 4B (Hersteller Pharmacia, Uppsala) gebunden. Nach Beendigung der Inkubation wurden die beads durch Sedimentation vom Reaktionsgemisch abgetrennt. Hierauf wurde mit 10 mg Natriumborhydrid (NaBH4) reduziert und 0,9 mg cis-3-Hydroxyprolin erhalten.

Beispiel 2

1 mg trans-3-Hydroxyprolin wurde in 10 ml 0,25 molarem Phosphatpuffer (pH 8,0) aufgelöst. Es wurde 1 mg Catalase (65 000 U/mg, Hersteller: Boehringer, Mannheim) zugegeben.

In der so erhaltenen Lösung wurden 1 ml Kügelchen aus Affi-Gel-10 (Hersteller Bio-Rad) mit denen 1 mg D-Aminosäureoxidase und 0,5 mg L-Aminosäureoxidase gekoppelt worden wind, suspendiert.

Die Kupplung der beiden Enzyme an die Kügelchen wurde wie folgt ausgeführt:

Der vorgequollene Gelkuchen wurde nach den Angaben des Herstellers gewaschen und mit 10 ml einer 0,1 molaren Natriumbicarbonatpufferlösung (pH 8,0) sowie mit der Enzymlösung (10 ml Bicarbonatpuffer und 1 mg jedes Enzyms) bei Raumtemperatur 4 Stunden lang in einem Schüttler vermischt. Die Blockierung der verbleibenden aktiven Gruppen wurde mit 10 ml eines 1 molaren tris-Puffers (pH 8,0) und 4 stündigem Inkubieren bei Raumtemperatur ausgeführt. Das Gel wurde dann in einem Büchner-Trichter mit destilliertem Wasser (drei mal das Volumen des Gels) und einer 0,025 molaren Phosphatpufferlösung (pH 8,0) gewaschen.

Diese Suspension der immobilisierten Enzyme und des Substrates (trans-3-Hydroxyprolin) wurde 24 Stunden lang bei 37°C unter Durchleiten von Sauerstoff in einem Abzug (um die Entzündungsgefahr zu verringern) inkubiert.

Hierauf wurden die Kügelchen (beads) absitzen gelassen und die überstehende Flüssigkeit abgesaugt.

Zur überstehenden Flüssigkeit wurden 5 mg NaBH4 zugegeben und 30 minuten lang bei Raumtemperatur inkubiert.

So wurden 91 % des eingesetzten trans-3-Hydroxyprolin in cis-3-Hydroxyprolin umgewandelt.

Beispiel 3

1 mg trans-3-Hydroxyprolin wurde in 10 ml 0,025 molarem Phosphatpuffer (ph 8,0) aufgelöst und dann 1 mg Catalase (65 000 U/mg) zugegeben.

In die so erhaltene Lösung wurden 1 ml Kügelchen aus mit Cyanbromid aktivierter Sepharose 4B (Hersteller: Pharmacia, Uppsala) an die D-Aminosäureoxidase (1 mg) und L-Aminosäureoxidase (0,5 mg) gebunden waren, suspendiert.

Das Koppeln der beiden Enzyme am Träger erfolgte wie folgt:

Das Gel (als Pulver) wurde in 0,01 molarer Salzsäure quellen gelassen und schließlich mit dem Ligandenpuffer, einem 0,025 molaren Phosphatpuffer (pH 8,4) in der Kälte (4°C) gewaschen. Die die beiden Enzyme enthaltende Ligandenlösung wurde dem suspendierten Gel zugegeben und 4 Stunden lang bei Raumtemperatur inkubiert.

Hierauf wurde 4 Stunden lang bei Raumtemperatur mit 1 molarem tris-Puffer (pH 8,0) inkubiert, um die verbleibenden, aktiven Gruppen zu blockieren. Nach diesem Arbeitsschritt wurde das Gel mit der für die Enzymreaktion verwendeten Pufferlösung gewaschen.

Die immobilisierten Enzyme wurden mit dem Substrat 24 Stunden lang bei 37°C unter Sauerstoffdurchleitung in einem Abzug inkubiert.

Nach 24 Stunden wurden die Kügelchen (beads absetzen gelassen und überstehende Flüssigkeit für die Reduktion abgetrennt. Zur Ausführung der Reduktion wurden der überstehenden Flüssigkeit 5 mg NaBH4 zugegeben und 30 minuten lang bei Raumtemperatur inkubiert. Auf diese Weise wurden 90 % des eingesetzten trans-3-Hydroxyprolins als cis-3-Hydroxyprolin wiedergewonnen.

Beispiel 4

1 mg trans-3-Hydroxyprolin wurde in 10 ml 0,025 molarem Phosphatpuffer (pH 8,0) aufgelöst. In die Lösung wurden 2 ml von Kügelchen aus Affi-Gel-10 (Hersteller: Bio-Rad), an die 1 mg D-Aminosäureoxidase, 0,5 mg L-Aminosäureoxidase und 1 mg Catalase gekoppelt waren, suspendiert.

Das Koppeln der drei Enzyme an die Kügelchen erfolgte wie in Beispiel 2 angegeben.

Die Oxidation und die Reduktion wurde ebenfalls wie in Beispiel 2 angegeben, ausgeführt.

Nach diesem Verfahren wurden 85 % des eingesetzten trans-3-Hydroxyprolins in cis-3-Hydroxyprolin umgewandelt.

### Beispiel 5

in 100 ml 0,025 molarem Phosphatpuffer mit pH 8,0 wurden 1 mg trans-3-Hydroxyprolin aufgelöst. In 5 ml 0,025 molarem Phosphatpuffer (pH 8,0) kwurden 1 mg Catalase, 1 mg D-Aminosäureoxidase und 0,5 mg L-Aminosäureoxidase aufgelöst und die Lösung in einem Dialysebeutel (hergestellt aus einem üblichen Dialyseschlauch) eingefüllt.

Der Dialysebeutel wurde dann in die die Ausgangsverbindung (trans-3-Hydroxyprolin) enthaltene Pufferlösung gegeben.

Hierauf wurde unter Rühren und Sauerstoffdurchleiten 24 Stunden bei 37°C unter einem Abzug inkubiert.

Nach beendeter Inkubation wurde der die Enzyme enthaltende Dialysebeutel entfernt (und einer weiteren Oxidationsstufe zugeführt) und die erhaltene Lösung wie in Beispiel 2 angegeben, reduziert.

Es wurden 82 % das eingesetzten trans-3-Hydroxyprolins in cis-3-Hydroxyprolin umgewandelt.

### Beispiel 6

1 mg trans-3-Hydroxyprolin, 1 mg Catalase, 1 mg D-Aminosäureoxidase und 0,5 mg L-Aminosäureoxidase wurden in 10 ml eines 0,05 molaren Phosphatpuffers (pH 8,0) aufgelöst und in einen Dialyseschlauch gegeben.

Der Dialyseschlauch wurde in 100 ml des oben erwähnten Puffers gegeben und die Inkubation wie in Beispiel 5 angegeben unter Rühren ausgeführt.

Es wurden 80 % des als Ausgangsverbindung eingesetzten trans-3-Hydroxyprolins in cis-33-Hydroxyprolin umgewandelt.

### Beispiel 7

Falls das als Ausgangsverbindung eingesetzte trans-3-Hydroxyprolin nicht racemisch ist, was beim Säugetiersystem der Fall sein kann, dann kann bei den oben erwähnten Ausführungsbeispielen 2 bis 6 auch ein Verhältnis zwischen L-Amonisäureoxidase zu D-Aminosäureoxidase von 1:10 verwendet werden.

### Beispiel 8

Die in den Beispielen 2 bis 7 beschriebenen Reaktionen können auch in Gegenwart anderer, natürlich vorkommender Aminosäuren, Iminosäuren, wie z.B. in Hydrolysaten ausgeführt werden.

Je nach der Konzentration und der Natur der Aminosäuren werden 77 bis 90 % von trans-3-Hydroxyprolin in cis-3-Hydroxyprolin umgewandelt, wenn die Verfahrensweisen der Beispiele 1 bis 7 angewendet werden.

### Beispiel 9

Nach der Vorschrift von J. Häusler (Liebigs Annalen der Chemie, 1981, 1073 - 1088) wurde trans-3-Hydroxyprolin hergestellt und als Kristalle gewonnen. Das so hergestellte trans-3-Hydroxyprolin kann nach den Vorschriften der Beispiele 1 bis 8 in cis-3-Hydroxyprolin umgewandelt werden.

Nachstehend werden einige Vorschriften für die Herstellung von Substraten, wie sie für die Durchführung der in den Beispielen angegebenen Verfahren eingesetzt werden können, angegeben.

### Vorschrift 1

Synthese von trans-3- und cis-3-Hydroxyprolin durch Umsetzen von Acrolein mit Amminomalonsäure:

Zu einer Lösung von 2,2 g Natriummetabisulfit in 30 ml 1 molarem Natriumacetat (pH 4,5) wurden 1,4 ml frisch destilliertes Acrolein unter Rühren bei einer Temperatur unter 30°C zugegeben. Nach 45 Minuten wurden 1,19 g Aminomalonsäure, aufgelöst in 30 ml 1 molarer Kaliumacetatpufferlösung (pH 4,5), zugegeben und 18 Stunden lang bei bis zu 60°C stehen gelassen. Hierauf wurde eine weitere Äquivalentmenge des, wie oben beschrieben, hergestellten Acrolein-Bisulfit-Adduktes in lösung (30 ml) zugegeben. Das Gemisch wurde bei 60°C 40 Stunden lang stehen gelassen. Man erhielt so 31 % trans-3-Hydroxyprolin und 63 % cis-3-Hydroxyprolin.

Die so erhaltene Mischung der beiden 3-Hydroxyproline konnte für die Verfahren der Beispiele 1 bis 8 nicht eingesetzt werden. Es wurde daher noch die folgende Vorbehandlung ausgeführt:

Mit dem oben erhaltenen Reaktionsprodukt wurden 50 g Aktivkohle vermischt und nach intensiver Durchmischung bei 2000 g zentrifugiert. Die überstehende Flüssigkeit wurde auf einen Dowex W 50 Ionentauscher in der H$^+$-Form aufgegeben und die Aminosäuren fraktioniert eluiert.

Nach dem Eindampfen konnten die Aminosäuren für die enzymatische Behandlung (Beispiele 1 bis 8) verwendet werden, nachdem sie in einem 0,025 molarem Phosphatpuffer mit pH 8,0 aufgelöst worden sind.

### Vorschrift 2

Gewinnung von trans-3-Hydroxyprolin aus Kadavern:

100 g Säugetierkadaver (Mäuse und Ratten) wurden während 16 Stunden bei 105°C mit 500 ml 6 normaler Salzsäure einer sauren Hydrolyse unterzogen.

Das erhaltene Gemisch wurde filtriert und die primären Aminosäuren nach der Vorschrift von F. Irreverre, K. Morita, A. V. Robertson und B. Witkop (J.Amer.Chem.Soc. 85, 2824 (1963) einer Trinitrophenylierung unterzogen. Nach dem Abfiltrieren der primären Aminosäuren zur Abtrennung deselben wurden aus dem verbleibenden Reaktionsgemisch trans-3- und cis-3-Hydroxyprolin durch Reaktion mit Aktivkohle (10 g/100 ml Hydrolysat), gefolgt von Filtration oder Zentrifugieren bei 2000 x g und Aussalzen auf einem IOnenaustauscher (Dowex W 50) in der $H^+$-Form und fraktionelle Eluierung durch einen Ammoniumgradienten mit 0,1 bis 0,2 M durch Flüssigkeits-Chromatografie gewonnen.

## Vorschrift 3

Abtrennung von trans-3- und cis-3-Hydroxyprolin aus handelsüblichen Champignons:

5 g feuchter Champignons werden bei 105°C 16 Stunden lang mit 20 ml 6 normaler Salzsäure hydrolisiert. Nach beendeter Hydrolyse wurden die primären Aminosäuren zu ihrer Abtrennung einer Trinitrophenylierungsreaktion unterzogen. Nach der Filtration und einer Aktivkohlenbehandlung des Produktes (wie in Vorschrift 2 angegeben) wurde das Reaktionsgemisch durch Radialchromatografie nach dem Eindampfen zur Trockene abgetrennt.

Das cis-3-Hydroxyprolin wurde abgetrennt und verwertet und das trans-3-Hydroxyprolin wurde wie in den obigen Beispielen angegeben, in cis-3-Hydroxyprolin umgewandelt.

## Vorschrift 4

Abtrennung von trans-3-Hydroyprolin aus Kadavern:

100 g Säugetierkadaver (Mäuse) wurden bei 105°C 16 Stunden lang mit 500 ml 6 normaler Salzsäure hydrolysiert.

Das Gemisch wurde filtriert und die primären Aminosäuren einer Trinitrophenylierungsreaktion unterzogen, wie dies von Irreverre und Mitarbeitern a.a.O. beschrieben ist.

Nach dem Abfiltrieren der primären Aminosäuren wurde das Reaktionsgemisch mit Aktivkohle (100 g/100 ml Hydrolysat) versetzt und hierauf filtriert oder bei 2000 g zentrifugiert.

Die Abtrennung und Isolierung von trans-3-Hydroxyprolin wurde nach Eindampfen zur Trockene durch Flüssigkeit-Chromatografie ausgeführt.

## Patentansprüche

1. Chemische Verbindung, bestehend aus cis-3-Hydroxyprolin zur Anwendung in einem Verfahren zur therapeutischen Behandlung des menschlichen oder tierischen Körpers.

2. Chemische Verbindung nach Anspruch 1, zur Anwendung bei der Bekämpfung von Verdickungen der Basalmembran.

3. Arzneimittel, dadurch gekennzeichnet, daß es aus cis-3-Hydroxyprolin besteht.

4. Pharmazeutische Zubereitungen, dadurch gekennzeichnet, daß sie als Wirkstoff cis-3-Hydroxyprolin sowie einen inerten pharmazeutischen Träger enthalten.

5. Verwendung von cis-3-Hydroxyprolin bei der Herstellung eines Arzneimittels zur Verwendung bei der Bekämpfung von Verdickungen der Basalmembran.

6. Verfahren zur Herstellung von cis-3-Hydroxyprolin dadurch gekennzeichnet, daß man trans-3-Hydroxyprolin zur entsprechend 1.2-Dehydroverbindung oxidiert und die so erhaltene 1.2-Dehydroverbindung reduziert.

7. Verfahren nach Anspruch 6, dadurch gekennzeichnet, daß man die Oxidation mit Hilfe eines Enzyms oder eines Enzymgemisches ausführt.

8. Verfahren nach einem der Ansprüche 6 und 7, dadurch gekennzeichnet, daß man als Enzym D-Aminosäureoxidase (E.C. 1.4.3.3) in Kombination mit L-Aminosäureoxidase (E.C. 1.4.3.2) und Catalase einsetzt.

9. Verfahren nach einem der Ansprüche 7 und 8, dadurch gekennzeichnet, daß man das Enzym und/oder das Enzymgemisch in immobilisierter Form in das Reaktionsmedium einbringt.

10. Verfahren nach einem der Ansprüche 7 bis 9, dadurch gekennzeichnet, daß man das Enzym bzw. die Enzyme in das Reaktionsmedium in einer Dialysemembran eingeschlossen, z.B. in einem Dialyseschlauch eingeschlossen, in das Rekationsmedium einbringt.

11. Verfahren nach einem der Ansprüche 7 bis 9, dadurch gekennzeichnet, daß man Enzym bzw. die Enzyme auf Kunstharzkügelchen (beads) immobilisiert in das Reaktionsmedium einbringt.

12. Verfahren nach Anspruch 11, dadurch gekennzeichnet, daß man Kunstharzkügelchen aus mit Cyanbromid aktivierter Sepharose 4B oder aus Affi-Gel-10 verwendet.

13. Verfahren nach einem der Ansprüche 6 bis 12, dadurch gekennzeichnet, daß man die Reduktion der 1.2-Dehydroverbindung zum cis-3-Hydroxyprolin mit einer komplexen Wasserstoffverbindung, insbesonders mit Natriumborhydrid ($NaBH_4$), ausführt.

14. Verfahren nach einem der Ansprüche 6 bis 13, dadurch gekennzeichnet, daß man als Ausgangsprodukt ein Gemisch aus trans- und cis-3-Hydroxyprolin einsetzt.

15. Verfahren nach einem der Ansprüche 6 bis 14, dadurch gekennzeichnet, daß man als Ausgangsprodukt ein Gemisch von bei der Kadaververwertung nach der Hydrolyse anfallenden Aminosäuren verwendet.